# EUROPEAN PATENT APPLICATION

(11) **EP 3 747 435 A1**
(43) Date of publication of application: **09.12.2020**
(21) Application number: 19747673.2
(22) Date of filing: 28.01.2019
(51) Int. Cl.: A61K 31/353, A61K 9/00, A61P 3/10

(54) **PHARMACEUTICAL COMPOSITION FOR PREVENTING OR TREATING DIABETES COMPLICATIONS COMPRISING NOVEL CHRYSIN DERIVATIVE COMPOUND AS ACTIVE INGREDIENT**

(30) Priority: 31.01.2018 KR 20180012511
(71) Applicant: FRONTBIO INC., Chuncheon-si, Gangwon-do 24232 (KR)
(72) Inventor: LIM, Soon Sung, Chuncheon-si Gangwon-do 24278 (KR); HWANG, Seung Hwan, Namyangju-si Gyeonggi-do 12239 (KR); LEE, Soo Kyeong, Chuncheon-si Gangwon-do 24320 (KR); ZUO, Guang Lei, Chuncheon-si Gangwon-do 24252 (KR); KWON, Jeong Han, Goyang-si Gyeonggi-do 10359 (KR); HAN, Jong Woo, Hanam-si Gyeonggi-do 12947 (KR); HAN, Bok Nam, Chuncheon-si Gangwon-do 24336 (KR); LEE, Ji Yeon, Ansan-si Gyeonggi-do 15591 (KR); PARK, Kyeong Hee, Chuncheon-si Gangwon-do 24395 (KR); LEE, Hyun Kyung, Seoul 05233 (KR); SA, Yun Ji, Guri-si Gyeonggi-do 11931 (KR); LEE, Ji Hyun, Seoul 02575 (KR)
(74) Representative: Pfenning, Meinig & Partner mbB
(86) International application number: PCT/KR2019/001158
(87) International publication number: WO 2019/151732

(57) **Abstract**

Disclosed is a pharmaceutical composition for preventing or treating diabetes complications containing a novel chrysin derivative compound as an active ingredient, and more specifically, a pharmaceutical composition for preventing or treating diabetes complications containing, as an active ingredient, a novel chrysin derivative compound that is capable of preventing or treating diabetes complications due to the ability thereof to inhibit the formation of an advanced glycation end-product (AGE).

## Description

### [Technical Field]

The present invention relates to a pharmaceutical composition for preventing or treating diabetes complications containing a novel chrysin derivative compound as an active ingredient, and more specifically, a pharmaceutical composition for preventing or treating diabetes complications containing, as an active ingredient, a novel chrysin derivative compound that is capable of preventing or treating diabetes complications due to the excellent ability thereof to inhibit the formation of an advanced glycation end-product (AGE).

### [Background Art]

Diabetes mellitus (DM) is a progressive disease that is often associated with obesity and is characterized by both insulin deficiency and insulin resistance. The increased fasting and postprandial blood glucose levels cause patients to experience acute and chronic complications (micro- and macro-vascular diseases) that may result in blindness, kidney failure, heart disease, strokes and amputations. Improvements in blood glucose control have been shown to lower the risk of these diabetes complications.

Advanced treatment strategies are required to maintain blood glucose control due to the progressive nature of these diseases. There are two types of diabetes mellitus, namely, type 1 diabetes mellitus (or pediatric diabetes or insulin-dependent diabetes mellitus (IDDM)), and type 2 diabetes (or adult diabetes or non-insulin-dependent diabetes mellitus (NIDDM)). Patients of type 1 diabetes mellitus are completely deficient in insulin due to the immunological destruction of pancreatic β cells that synthesize and secrete insulin. Type 2 diabetes mellitus is more complicated in etiology and is characterized by relative insulin deficiency, decreased insulin action and insulin resistance. Early-onset NIDDM or maturity-onset diabetes of the young (MODY) shares many characteristics with NIDDM, which is the most common type that develops in middle age (Rotter et al., 1990). Inheritance with an elucidated mechanism (autosomal dominant) was observed in MODY. At least three completely different mutations have been identified in the MODY family (Bell et al., 1996).

Meanwhile, diabetes complications include diabetic neuropathy, diabetic nephropathy, diabetic myocardial infarction, diabetic retinopathy, diabetic cataracts, vascular diabetes complications or diabetic ulcers, the main cause of which is known to be accumulation of excess sorbitol due to the activity of aldose reductase of the polyol pathway. Therefore, it is known that the inhibition of aldose reductase plays an important role in the treatment and prevention of diabetes complications. Aldose reductase inhibitors developed to date, such as zopolrestat, ponalrestat, sorbinil, tolrestat, fidarestat, ranirestat and epalrestat, have been reported to prevent and delay diabetes complications in various animal experiments.

However, zopolrestat and ponalrestat exhibited low efficacy in clinical trials, and side effects such as hypersensitivity of sorbinil and liver dysfunction of tolrestat made the development thereof stopped. Currently, clinical trials on ranirestat and fidarestat are being conducted in Japan and the United States. Epalrestat is not approved by the United States Food and Drug Administration (FDA), but was approved only by Japan in 1992, and is commercially available at present.

In addition, sorbitol accumulation causes conversion of sorbitol to fructose by a sorbitol dehydrogenase, so that a high concentration of fructose binds to proteins, ultimately accelerating the formation of an advanced glycation end-product (AGE). Such an AGE is known to attack biological tissues and cells to thereby accelerate aging, and is involved in the onset of various diseases including diabetes, heart disease and cancer. In addition, when the advanced glycation end-product (AGE) is formed, receptors (RAGE: receptor of AGEs) that can bind to the advanced glycation end-product (AGE) are formed on the cell membrane of blood vessel walls or lymphocytes. When the advanced glycation end-product (AGE) binds to the receptors, various immune factors associated with inflammation are activated, thus inducing or worsen chronic diseases.

Meanwhile, chrysin is contained in the young leaves of cottonwood (*Populus nigra* L.), the heart wood of *Pinus parviflora* Sieb. et Zucc. (five-leaf pine), the bark of *Cormus tschonoskii* Maxim, as the glycoside sylrgin or the like. Chrysin is commonly known to have an antioxidant effect and is used for analgesics and the like.

However, no prior art has been reported on the use of the novel chrysin derivative compound of the present invention for the prevention or treatment of diabetes complications.

### [Disclosure]

### [Technical Problem]

Accordingly, in the present invention, research was conducted on an active substance having an effect of preventing or treating diabetes complications. As a result, it was found that a novel chrysin derivative compound has inhibitory activity on the formation of an advanced glycation end-product (AGE). Accordingly, it is an object of the present invention to provide a pharmaceutical composition for preventing or treating diabetes complications containing, as an active ingredient, a novel chrysin derivative compound that can be used to prevent or treat diabetes complications due to the ability thereof to inhibit the formation of an advanced glycation end-product (AGE) .

### [Technical Solution]

In accordance with one aspect of the present invention, the above and other objects can be accomplished by the provision of a pharmaceutical composition for preventing or treating diabetes complications containing a chrysin derivative compound represented by the following Formula 1 as an active ingredient: 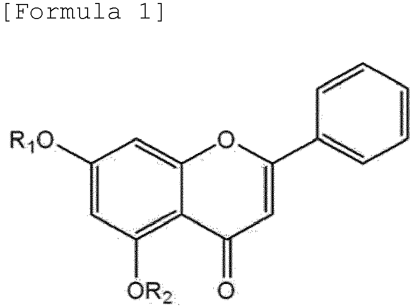 wherein
R₁ is selected from the group consisting of C₁₋₄ alkyl, C₁₋₆ alkenyl and -COR₃;
R₂ is selected from the group consisting of H, halogen, C₁₋₄ alkyl and -COR₄; and
R₃ and R₄ are each independently C₁₋₄ alkyl.

In another aspect of the present invention, provided is a pharmaceutical formulation containing the pharmaceutical composition.

### [Advantageous Effects]

The novel chrysin derivative compound provided by the present invention is useful for the prevention or treatment of diabetes complications due to the efficacy thereof to inhibit the formation of an advanced glycation end-product (AGE).

### [Description of Drawings]

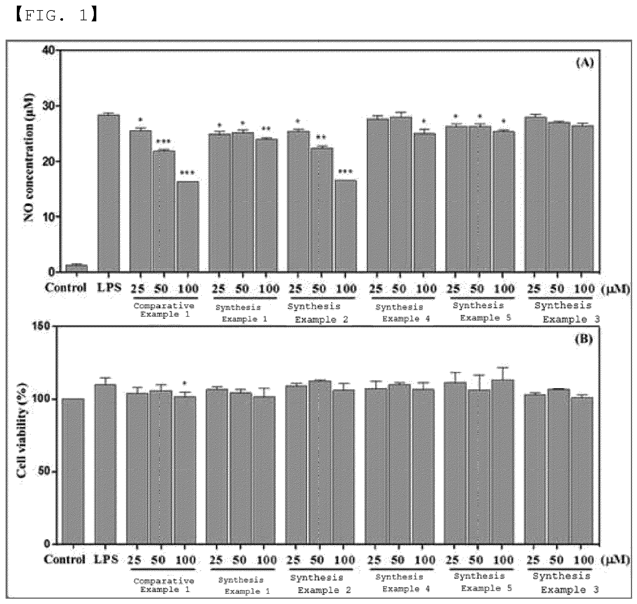

FIG. 1 shows the result of a determination of the effect of chrysin and a derivative thereof on NO production (A) and cell survival (B) in RAW 264.7 cells as an embodiment of the present invention.

### [Best mode]

The definitions listed below include definitions of various terms used to describe the present invention. Such definitions are applied throughout this specification individually or as part of a term including the same, unless specified otherwise.

As used herein, the term "halogen" means fluorine, chlorine, bromine or iodine, unless specified otherwise.

As used herein, the term "alkyl" refers to a saturated, straight or branched hydrocarbon radical represented by CₙH₂ₙ₊₁, unless mentioned otherwise, and specifically refers to a saturated, straight or branched hydrocarbon radical including 1 to 4 carbon atoms. Examples of these radicals include, but are not limited to, methyl, ethyl, propyl, isopropyl, n-butyl, and t-butyl. For example, as used herein, the term "C₁₋₄ alkyl", unless mentioned otherwise, means a straight or branched hydrocarbon residue having 1 to 4 carbon atoms. Examples thereof include, but are not limited to, methyl, ethyl, propyl, isopropyl, n-butyl, sec-butyl, t-butyl and the like.

As used herein, the term "alkenyl", unless mentioned otherwise, refers to a monovalent group derived from an unsaturated, straight or branched hydrocarbon moiety having at least one carbon-carbon double bond, and specifically refers to an unsaturated, straight or branched monovalent group containing 2 to 6 carbon atoms. Examples thereof include, but are not limited to, ethenyl, propenyl, butenyl and 1-methyl-2-buten-1-yl radicals.

Hereinafter, the present invention will be described in more detail.

The present invention relates to a novel chrysin derivative compound of the following Formula 1 and uses thereof, and more particularly to a pharmaceutical composition for preventing or treating diabetes complications containing a chrysin derivative compound as an active ingredient. The novel chrysin derivative compound of the present invention is capable of preventing or treating diabetes complications owing to the effects of efficiently controlling blood glucose and inhibiting the formation of an advanced glycation end-product (AGE).

In one aspect, the present invention provides a pharmaceutical composition for preventing or treating diabetes complications containing a chrysin derivative compound represented by the following Formula 1 as an active ingredient:

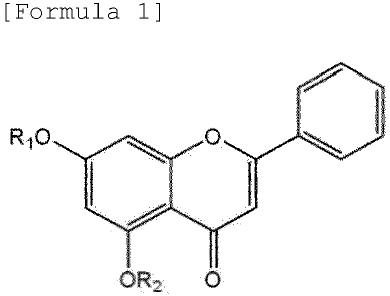

wherein
R₁ is selected from the group consisting of C₁₋₄ alkyl, C₁₋₆ alkenyl and -COR₃;
R₂ is selected from the group consisting of H, halogen, C₁₋₄ alkyl and -COR₄; and
R₃ and R₄ are each independently C₁₋₄ alkyl.

In another embodiment of the present invention, in the compound represented by Formula 1, R₁ is -COR₃, R₂ is selected from the group consisting of H, -CH₃ and -COCH₃, and R₃ is C₁₋₄ alkyl.

In another embodiment of the present invention, in the compound represented by Formula 1, R₁ is -COCH₃.

Preferred examples of the compound of Formula 1 according to the present invention include, but are not limited to, the following:
7-*O*-acetyl chrysin;
5,7-di-*O*-acetyl chrysin;
7-*O*-prenyl chrysin;
7-*O*-methoxy chrysin; and
5,7-di-*O*-methoxy chrysin.

The compound represented by Formula 1 contained in the pharmaceutical composition of the present invention inhibits the formation of an advanced glycation end-product (AGE), so the pharmaceutical composition can be useful for the prevention or treatment of various diseases associated therewith.

In one embodiment of the present invention, the compound is preferably 5,7-di-*O*-acetyl chrysin, which has an excellent inhibitory effect on the formation of an advanced glycation end-product (AGE), but is not limited thereto.

In one embodiment of the present invention, the diabetes complication includes at least one selected from the group consisting of diabetic neuropathy, diabetic nephropathy, diabetic myocardial infarction, diabetic retinopathy, diabetic cataracts, and diabetic ulcers, but is not limited thereto.

As used herein, the term "prevention" refers to any action that inhibits or delays the onset of diabetes complications by administering the pharmaceutical composition of the present invention to a subject.

As used herein, the term "treatment" refers to any action that ameliorates or positively affects symptoms of diabetes complications by administering the pharmaceutical composition of the present invention to a subject.

In another aspect, the present invention provides a pharmaceutical formulation containing the pharmaceutical composition.

The pharmaceutical formulation of the present invention may be selected from various forms for oral administration such as tablets, pills, powders, capsules, syrups or emulsions, or forms for parenteral administration such as intramuscular, intravenous or subcutaneous administration, for example, injections. The pharmaceutical formulation of the present invention is preferably a form for oral administration.

In addition, the pharmaceutical formulation may be prepared in accordance with a conventional method by adding at least one selected from the group consisting of ordinary non-toxic pharmaceutically acceptable additives, for example, carriers, adjuvants, and excipients, in addition to the active ingredient.

The excipients that can be used in the pharmaceutical formulation of the present invention may include sweeteners, binders, solubilizers, sub-solubilizers, wetting agents, emulsifiers, isotonic agents, adsorbents, disintegrants, antioxidants, preservatives, lubricants, fillers, fragrances and the like, but are not limited thereto. Examples of the excipients that can be used include lactose, dextrose, sucrose, mannitol, sorbitol, cellulose, glycine, silica, magnesium aluminum silicate, starch, gelatin, tragacanth rubber, alginic acid, sodium alginate, methylcellulose, sodium carboxymethyl cellulose, water, ethanol, polyethylene glycol, polyvinylpyrrolidone, sodium chloride, calcium chloride, orange essence, strawberry essence, vanilla flavor and the like.

When the pharmaceutical formulation of the present invention is a form for oral administration, examples of the carriers that can be used include cellulose, calcium silicate, corn starch, lactose, sucrose, dextrose, calcium phosphate, stearic acid, magnesium stearate, calcium stearate, gelatin, talc and the like, but are not limited thereto.

When the pharmaceutical preparation of the present invention is an injection form, the carrier may be water, saline, an aqueous glucose solution, an aqueous pseudo-sugar solution, alcohol, glycol, ether, oil, fatty acid, fatty acid ester, glyceride and the like, but is not limited thereto.

For the use of the compound according to the invention as a pharmaceutical, the latter is prepared in the form of a pharmaceutical formulation, which contains, in addition to the active ingredient for oral or parenteral administration, an appropriate pharmaceutical organic or inorganic inert carrier substance, for example water, gelatin, gum arabic, lactose, starch, vegetable oil, polyalkylene glycol and the like. The pharmaceutical formulation may be present as a solid form, for example, a tablet, dragee, suppository or capsule, or as a liquid form, for example, a liquid, suspension or emulsion. In addition, these optionally contain an additive, such as a preservative, stabilizer, wetting agent or emulsifier, a salt or a buffer for changing osmotic pressure.

For parenteral administration, an injection solution or suspension is particularly preferred.

As a carrier system, a surfactant additive such as a bile acid salt or animal or plant phospholipid, or a mixture thereof, and a liposome or ingredient thereof can also be used.

For oral administration, a tablet, dragee or capsule containing talc and/or a hydrocarbon vehicle or binder, for example, lactose, corn, or potato starch, is particularly suitable. In addition, a liquid form, for example, juice supplemented with a sweetener, may be administered.

In addition, the dose of the compound of Formula 1 according to the present invention administered to the human body is generally within the range of 0.1 mg/day to 2,000 mg/day based on an adult patient weighing 70 kg. The compound according to the present invention may be administered once or several times a day in a portionwise manner. However, the dose may vary depending on the patient's health status, age, weight and gender, and the dosage form and disease level (severity), and accordingly, the scope of the present invention is not limited to the dose described above.

### [Mode for Invention]

Hereinafter, the present invention will be described in more detail with reference to examples. However, the examples are provided only for illustration of the present invention, and should not be construed as limiting the scope of the present invention.

### Example 1

**[Table 1]**

| Item | Chemical structure | General name |
|---|---|---|
| | | IUPAC name |
| Comparative Example 1 | 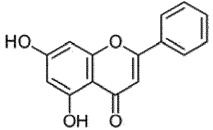 | Chrysin |
| | | 5,7-dihydroxy-2-phenyl-4H-chromen-4-one |
| Synthesis Example 1 | 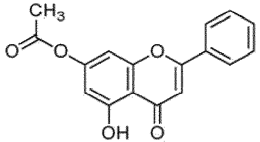 | 7-*O*-acetyl chrysin |
| | | 5-hydroxy-4-oxo-2-phenyl-4H-chromen-7-yl acetate |
| Synthesis Example 2 | 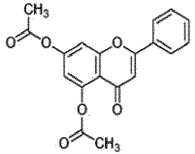 | 5,7-di-*O*-acetyl chrysin |
| | | 4-oxo-2-phenyl-4H-chromen-5,7-diyl diacetate |
| Synthesis Example 3 | 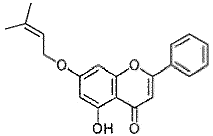 | 7-*O*-prenyl chrysin |
| | | 5-hydroxy-7-((3-methyl-2-buten-1-yl)oxy)-2-phenyl-4H-chromen-4-one |
| Synthesis Example 4 | 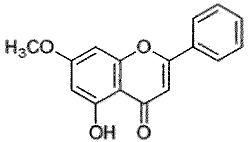 | 7-*O*-methoxy chrysin |
| | | 5-hydroxy-7-methoxy-2-phenyl-4H-chromen-4-one |
| Synthesis Example 5 | 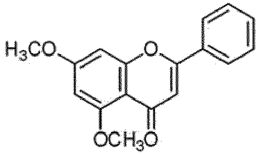 | 5,7-di-*O*-methoxy chrysin |
| | | 5,7-dimethoxy-2-phenyl-4H-chromen-4-one |

### [Synthesis Examples 1 and 2] Synthesis of 7-O-acetyl chrysin and 5,7-di-O-acetyl chrysin

Acetic anhydride (10 mM) was added dropwise to a solution containing 50 mL of pyridine and 10 mM of chrysin. The resulting mixture was allowed to react for 2 hours while being stirred at room temperature, and then the solvent was removed at 40°C using a rotary evaporator.

The residue was dissolved in methylene chloride (MC), washed 3 times with 1M HCl and then neutralized with a saturated sodium bicarbonate solution and water. The organic phase was separated, dried over MgSO₄ and concentrated under vacuum. The residue was eluted with MC/MeOH (10:0 to 9.5:1.5, v/v) to obtain 7-*O*-acetyl chrysin (7-OA) and 5,7-di-*O*-acetyl chrysin.

### [Synthesis Example 3] Synthesis of 7-O-prenyl chrysin

Chrysin (10 mM), prenyl bromide (2.2 mM) and anhydrous K₂CO₃ (3.7 mM) were added to anhydrous acetone (70 mL) and then the resulting mixture was refluxed at 65°C for 8 hours.

*The solvent was removed from the mixture at 40°C using a rotary evaporator. The residue was dissolved in ethyl acetate and then was purified by silica gel column chromatography with hexane/ethyl acetate (9:1:2:1, v/v) to obtain 7-*O*-7-*O*-prenyl chrysin.

### [Synthesis Examples 4 and 5] Synthesis of 7-O-methoxy chrysin and 5,7-di-O-methoxy chrysin

2.54 g of chrysin (0.5 mmol) and 1.84 g of 1,8-diazabicyclo(5.4.0)undec-7-ene were mixed with 80 mL of DMC (dimethyl carbonate) and reacted at 90°C for 19 hours.

The reaction product was concentrated with methanol (240 ml), and the resulting concentrate was fractioned with ethyl acetate (200 ml) and 1N HCl (100 ml).

The organic fraction layer (ethyl acetate) was concentrated, neutralized with NaCl, dehydrated with Na₂SO₄, and then subjected to silica gel column chromatography to separate 7-*O*-methoxy chrysin and 5,7-di-O-methoxy chrysin.

### Experimental example 1

### (1) Analysis of hemoglobin-δ-gluconolactone with respect to formation of Amadori compound (initial stage)

Evaluation of the initial stage of protein glycosylation was determined by δ-gluconolactone assay (Rahbar et *al.,* 1999) . Briefly, fresh human blood (50 mg/mL) was cultured with glucose (144 mg/mL) in a phosphate buffer (pH 7.4) containing 0.2 g/L of NaN₃ under conditions of cancer sterilized at 37°C for 7 days.

In certain experiments, an indicated sample was added in a concentration range of 0.01 to 1 mM to a model system. Fluorescence of the sample was measured at maximum excitation and emission values of 355 nm and 460 nm, respectively. The test was conducted using aminoguanidine, a known inhibitor, as a positive control group.

### (2) Bovine serum albumin - methylglyoxal analysis with respect to AGE formation (intermediate stage)

Bovine serum albumin (50 mg/mL) was incubated with methylglyoxal (100 mM) in a sodium phosphate buffer (0.1 mM, pH 7.4) at 37°C for 24 hours in the presence of various concentrations of compounds (including a control group).

The dimethyl sulfoxide used to dissolve the sample was found to have no effect on the reaction. All reagents and samples were sterilized by filtration through 0.2 mm membrane filters. Fluorescence intensity was measured at an excitation wavelength of 355 nm and an emission wavelength of 460 nm using a luminescence spectrometer LS50B (Perkin-Elmer Ltd., Buckinghamshire, England) (Wu & Yen, 2005). The test was conducted using aminoguanidine as a positive control group. The concentration of each test sample showing 50% inhibitory activity (IC₅₀) was estimated from the least squares regression line of logarithmic concentrations plotted against the residual activity.

### (3) N-acetyl-glycyl-lysine-methyl ester D-ribose assay on crosslinking of AGEs (late stage)

This test was used to evaluate the ability of the sample to inhibit crosslinking of GK peptides in the presence of D-ribose using the method described by Rahbar et *al.* (1999). The GK peptide (26.7 mg/mL) was incubated with D-ribose (200 mg/mL) under aseptic conditions in sodium phosphate buffer (0.5 M, pH 7.4) at 37°C for 24 hours. The synthesized compound was added to the model system at a final concentration of 1 mM, excluding the aminoguanidine used at 10 mM and 50 mM. At the end of the incubation period, fluorescence intensity was measured at an excitation wavelength of 335 nm and an emission wavelength of 460 nm.

### Results of Experimental example 1

The effect of the chrysin derivative for each step on the inhibition of the formation of the advanced glycation end-product is shown in [Table 2].

**[Table 2]**

| Item | IC₅₀ (µM) | | |
|---|---|---|---|
| | Initial stage | Intermediate stage | Late stage |
| Comparative Example 1 | 17.41 | 24.96 | NI |
| Synthesis Example 1 | 23.54 | 21.61 | > 200 |
| Synthesis Example 2 | 0.26 | 0.91 | 22.33 |
| Synthesis Example 3 | NI | > 200 | > 200 |
| Synthesis Example 4 | NI | > 200 | NI |
| Synthesis Example 5 | 7.88 | 41.78 | 39.88 |
| Positive control group (Aminoguanidine ) | 109.74 | 136.79 | 1902.67 |

### (1) Results of the initial stage: the inhibitory effect of the chrysin derivative on the formation of the Amadori compound

The ability of chrysin and derivatives thereof to inhibit the formation of the Amadori compound was compared. The result showed that the initial inhibitory activity of Synthesis Example 1 and Synthesis Example 5 was 4.66 times and 13.92 times higher, respectively, than the positive control. Synthesis Example 2 showed the strongest inhibitory activity against the formation of the Amadori compound, which was 66.96 times and 422.07 times higher than the chrysin and positive control group. Synthesis Example 2 showed a dose-dependent inhibition rate of 1.76 to 55.13% at 0.0025 to 0.05 µg/mL.

### (2) Results of intermediate step: the effect of chrysin derivative on inhibition of the formation of AGEs

The inhibitory activity against AGE formation of chrysin and derivatives thereof was analyzed and the results are shown in Table 2. The AGE inhibitor, aminoguanidine (IC₅₀=136.79 µM) was used as a positive control group and chrysin showed 5.48 times higher activity than the positive control group.

Among the test compounds, Synthesis Example 1, Synthesis Example 2 and Synthesis Example 5 showed strong activity with IC₅₀ values of 21.61, 0.91 and 41.78 µM, respectively. In particular, Synthesis Example 2 showed an inhibitory activity of 6.79 to 93.42% at a low concentration of 0.05 to 0.5 µg/mL.

### (3) Results of later stage: the effect of the chrysin derivative on inhibition of AGE crosslinking

As shown in Table 2, Synthesis Example 2 and Synthesis Example 5 exhibited significant inhibitory activity against AGE crosslinking. Synthesis Example 2 and Synthesis Example 5 exhibited IC₅₀ values of 22.33 and 39.88 µM, respectively, while the positive control group showed a low IC₅₀ value (1902.67 µM).

Similarly, Synthesis Example 2 and Synthesis Example 5 can be regarded as potential AGE inhibitors due to the low IC₅₀ values in three stages of AGE formation. On the other hand, chrysin did not exhibit inhibitory activity at a concentration of 10.0 mg/mL.

### Experimental Example 2: Measurement of NO production and cell viability in RAW 264.7 cells

The cytotoxicity of chrysin and derivatives thereof in RAW 264.7 cells was investigated using a MTS assay kit. Cells (1.6 x 10⁴/well) were cultured in 96-well plates and treated with samples (10, 25 and 100 µM) for 12, 24, 48 and 72 hours. After culture, a MTS solution was incubated at 20 µL/well at 37°C in a humidified 5% CO₂ atmosphere for 90 minutes. The optical density at 490 nm was measured three times using an EL-800 universal microplate reader (Bio-Tek Instrument Inc., Winooski, USA). The cell viability of a untreated group was set at 100%. RAW 264.7 cells were seeded at a density of 4×10⁵ cells/well on a 12-well plate and incubated with LPS (1 µg/mL) and samples of various concentrations for 24 hours. The concentration of nitrogen oxide (NO) in the medium was measured using a Griess reagent system described by the manufacturer. The production of NO was measured at 570 nm using an EL-800 Universal microplate reader (Bio-Tek Instrument Inc., Winooski, USA) and compared with the nitrite standard calibration curve [21].

### Results of Experimental Example 2

The results of the anti-inflammatory effect of the chrysin derivative in RAW 264.7 cells are shown in [FIG. 1].

The effect of chrysin and derivatives thereof on LPS-induced inflammation in RAW 264.7 cells was investigated, and NO concentration was used as a biomarker indicating the degree of cell inflammation. FIG. 1A shows the effects of Comparative Example 1, Synthesis Example 1, Synthesis Example 2, Synthesis Example 4 and Synthesis Example 5 on NO production in LPS-derived RAW 264.7 cells. In addition, Synthesis Example 2 treatment inhibited NO in a concentration-dependent manner in LPS-induced RAW 264.7 cells, and the effect thereof was similar to that of Comparative Example 1. The cell viability effect of chrysin and derivatives thereof in RAW 264.7 cells was observed by MTS analysis. The concentration of NO in the supernatant increased after LPS treatment and the compound exhibited no cytotoxicity at a concentration of 25 to 100 µM after 24 hours (FIG. 1B).

FIG. 1 shows the effects of chrysin and derivatives thereof on NO production (A) and cell survival (B) in RAW 264.7 cells. The symbol "*" indicates a significant difference from the LPS group (* p<0.05, ** p<0.01, *** p<0.001). Data is expressed as mean ± standard error (SEM) (n=3) .

### Experimental Example 3: Solubility Analysis

The chrysin and derivatives thereof were dissolved in distilled water and sonicated for 1 hour to maximize solubility and incubated at 37°C. After sonication, undissolved samples were removed by centrifugation (7000g, 37°C, 5 min). The concentration of the sample was analyzed by HPLC by diluting the supernatant with methanol and filtering through a 0.45 µm disposable syringe filter (Advantec, Dublin, CA, USA).

### Results of Experimental Example 3

Table 3 shows comparison in the solubility of the chrysin derivatives.

**[Table 3]**

| Item | Solubility in water (mM, 37°C) | Relative solubility |
|---|---|---|
| Comparative Example 1 | 0.030 | 1.00 |
| Synthesis Example 1 | 0.086 | 2.87 |
| Synthesis Example 2 | 0.265 | 8.83 |
| Positive control group | 0.030 | 1.00 |

Synthesis Example 1 and Synthesis Example 2 exhibited solubility in water of 0.086 and 0.265, which were respectively 2.87 times and 8.83 times higher than Comparative Example 1.

## Claims

1. A pharmaceutical composition for preventing or treating diabetes complications containing a chrysin derivative compound represented by the following Formula 1 as an active ingredient: 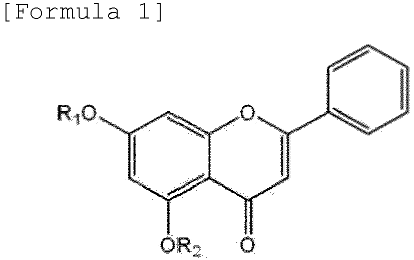
wherein R₁ is selected from the group consisting of C₁₋₄ alkyl, C₁₋₆ alkenyl and -COR₃;
R₂ is selected from the group consisting of H, halogen, C₁₋₄ alkyl and -COR₄; and
R₃ and R₄ are each independently C₁₋₄ alkyl.

2. The pharmaceutical composition according to claim 1, wherein R₁ is -COR₃, R₂ is selected from the group consisting of H, -CH₃ and -COCH₃, and R₃ is C₁₋₄ alkyl.

3. The pharmaceutical composition according to claim 2, wherein R₁ is -COCH₃.

4. The pharmaceutical composition according to claim 1, wherein the compound of Formula 1 is selected from the group consisting of the following compounds:
7-*O*-acetyl chrysin;
5,7-di-*O*-acetyl chrysin;
7-*O*-prenyl chrysin;
7-*O*-methoxy chrysin; and
5,7-di-*O*-methoxy chrysin.

5. The pharmaceutical composition according to claim 1, wherein the pharmaceutical composition inhibits formation of an advanced glycation end-product (AGE).

6. The pharmaceutical composition according to claim 1, wherein the diabetes complication comprises at least one selected from the group consisting of diabetic neuropathy, diabetic nephropathy, diabetic myocardial infarction, diabetic retinopathy, diabetic cataracts, and diabetic ulcers.

7. A pharmaceutical formulation comprising the pharmaceutical composition according to claim 1.

8. The pharmaceutical formulation according to claim 7, wherein the pharmaceutical formulation is a tablet, pill, powder, capsule, syrup or emulsion.

9. The pharmaceutical formulation according to claim 7, further comprising at least one selected from the group consisting of pharmaceutically acceptable carriers, adjuvants, and excipients.
